# FASCICULE DE BREVET EUROPEEN

(11) **EP 0 643 993 B1**
(45) Date de publication et mention de la délivrance du brevet: **09.12.1998**
(21) Numéro de dépôt: 94401979.3
(22) Date de dépôt: 06.09.1994
(51) Int. Cl.: B01J 27/053, B01J 27/02, B01J 21/08, B01J 35/10, C07C 2/62

(54) **Catalyseur d'alkylation d'isoparaffine C4-C5 par au moins une oléfine C3-C6**
Katalysator für die Alkylierung eines C4-C5 Isoparaffin mit mindestens einem C3-C6 Olefin
Catalyst for alkylation of a C4-C5 isoparaffin with at least one C3-C6 olefin

(30) Priorité: 10.09.1993 FR 9310896
(43) Date de publication de la demande: 22.03.1995
(73) Titulaire: INSTITUT FRANCAIS DU PETROLE, 92502 Rueil-Malmaison (FR)
(72) Inventeur: Joly, Jean François, F-75003 Paris (FR); Marcilly, Christian, F-78800 Houilles (FR); Benazzi, Eric, F-78360 Montesson (FR); Chaigne, Frédéric, F-85230 Bauvoir S/Mer (FR); Bernhard, Jean-Yves, F-91540 Mennecy (FR)

(56) Documents cités:
- EP-A- 0 067 467
- EP-A- 0 325 811
- EP-A- 0 393 356
- EP-A- 0 539 277
- EP-A- 0 542 612

## Description

La présente invention concerne un catalyseur comprenant de la silice et une phase acide comportant de l'acide sulfurique, et son utilisation en alkylation catalytique d'isobutane et/ou d'isopentane au moyen d'au moins une oléfine, qui permet d'obtenir au moins un produit par exemple dans le groupe constitué par les diméthylbutanes, les triméthylpentanes, les triméthylhexanes et les triméthylheptanes.

On sait que, pour alimenter les moteurs à combustion interne et à allumage commandé, et notamment les moteurs à taux de compression élevé, il est particulièrement intéressant de disposer de carburants à hauts indices d'octane, c'est-à-dire constitués essentiellement par des hydrocarbures paraffiniques fortement ramifiés. L'alkylation d'au moins une isoparaffine (isobutane et/ou isopentane) par au moins une oléfine contenant de 3 à 6 atomes de carbone par molécule permet d'obtenir de tels produits. Cette réaction nécessite la mise en oeuvre de catalyseurs très acides, dans le but notamment de réduire les réactions parasites telles que les réactions d'abstraction d'hydrure de l'oléfine et de polymérisation qui fournissent des hydrocarbures peu ramifiés de faible indice d'octane et des hydrocarbures insaturés, les réactions de craquage et les réactions de dismutation.

Les procédés existant pour la production d'hydrocarbures par alkylation de l'isobutane par les oléfines utilisent soit l'acide sulfurique soit l'acide fluorhydrique comme catalyseur. Dans ces procédés le catalyseur acide constitue une phase liquide qui est mise en contact avec le mélange isobutane-oléfine liquide pour former une émulsion. Ces procédés sont coûteux et posent d'importants problèmes vis-à-vis de la sécurité des personnes et de l'environnement. Afin de remédier à ces problèmes, des systèmes catalytiques différents des acides sulfurique et fluorhydrique en phase liquide ont été recherchés.

La demande de brevet européen EP-A-0 539 277 décrit un catalyseur contenant de la silice et une phase acide solide comprenant de l'acide sulfurique, la silice selon ladite demande de brevet est telle que son volume poreux est compris entre 0,005 et 1,5 cm³/g, et sa surface spécifique est comprise entre 0,01 et 1 500 m²/g. Ladite phase acide comprend éventuellement un additif choisi dans le groupe formé par H₃PO₄, B(OH)₃, BF₄H, FSO₃H, CF₃CO₂H, SbF₅, CF₃SO₃H et SO₃.
Les demandes de brevet européen EP-A-0 542 612 et EP-A-0 542 620 décrivent le même type de catalyseur, mais avec des supports différents : le support de catalyseur décrit dans EP-A-0 542 612 comprend au moins un oxyde sulfaté et le support de catalyseur décrit dans EP-A-0 542 620 est choisi parmi les résines, le charbon, les zéolithes, les oxydes tels que ZrO₂, T₂O₂, Al₂O₃, Fe₂O₃. HfO₂ et les catalyseurs de craquage usagés.

La présente invention concerne un catalyseur pour l'alkylation d'au moins une isoparaffine (isobutane et/ou isopentane) avec au moins une oléfine comprenant de 3 à 6 atomes de carbone par molécule. Plus précisément, l'invention concerne un catalyseur comprenant de la silice et une phase acide comportant de l'acide sulfurique, la silice ayant été imprégnée par ladite phase acide, sa préparation et son utilisation en alkylation catalytique d'isobutane et/ou d'isopentane en présence d'au moins une oléfine comportant de 3 à 6 atomes de carbone par molécule, ledit catalyseur étant caractérisé en ce qu'il est constitué essentiellement de particules de diamètre moyen compris entre 0,1 et 30 µm (1µm = 10⁻⁶m) de préférence entre 5 et 30 µm, et en ce que la silice, avant son imprégnation par ladite phase acide, a un volume poreux total compris entre 0,1 et 0,6 cm³/g, de préférence entre 0,1 et 0,4 cm³/g. La teneur pondérale dudit catalyseur en phase acide est généralement comprise entre 1 et 40%, de préférence entre 1 et 30%.

Le catalyseur selon la présente invention conduit à des performances catalytiques améliorées par rapport à celles décrites dans la demande de brevet européen EP-A-0539277.

La silice peut éventuellement contenir des impuretés comme par exemple les oxydes, les alcalins, les alcalino-terreux, les composés d'aluminium ou toute autre impureté connue de l'homme du métier, la quantité totale de ces impuretés n'excédant généralement pas 2 % en poids par rapport à la silice.

Le titre de l'acide sulfurique est avantageusement compris entre 90 et 100 % en poids, de préférence entre 97 et 100 % en poids et de manière encore plus préférée entre 98 et 100 % en poids.

Il est possible d'ajouter dans ladite phase acide, avant l'imprégnation, au moins un additif visant à améliorer les performances catalytiques.

L'additif est généralement choisi dans le groupe formé par H₃PO₄, B(OH)₃, CF₃SO₃H, HB(HSO₄)₄, BF₄H, FSO₃H, CF₃CO₂H, SbF₅, et SO₃ ; mais tout additif connu de l'homme du métier est envisageable. La teneur totale de la phase acide en additif(s) dépend de nombreux paramètres dont la nature de l'additif.

L'additif préféré est l'acide trifluorométhane sulfonique CF₃SO₃H. Lorsque l'acide trifluorométhane sulfonique est utilisé comme additif, le titre de l'acide trifluorométhane sulfonique est compris entre 98 et 100%, et la teneur de ladite phase acide en acide trifluorométhane sulfonique est comprise entre 0,1 et 50% (poids), de préférence entre 0,1 et 30% (poids) et de manière encore plus préférée entre 5 et 15% (poids).

La phase acide occupe entre 80 et 100% du volume poreux total de la silice, et de préférence entre 90 et 100% dudit volume poreux.

La silice est généralement telle que, avant son imprégnation par la phase acide, sa surface spécifique est comprise entre 0,1 et 1 500 m²/g. De plus, elle est généralement constituée essentiellement de particules de diamètre moyen compris entre 0,1 et 30 µm.

Le procédé de préparation du catalyseur selon l'invention comprend deux étapes. Dans une première étape la silice est calcinée à une température supérieure à 50 °C, de préférence supérieure à 80 °C et de manière encore plus préférée comprise entre 150 et 600°C, par exemple égale à environ 500° C. La durée de cette étape de calcination est habituellement comprise entre 10 minutes et 50 heures, de préférence entre 15 minutes et 25 heures. La calcination peut être effectuée en présence d'air sec ou de mélange air sec/azote, de débit compris entre 0,001 et 10 l/h/g, de préférence entre 0,1 et 5 l/h/g. La seconde étape consiste en l'imprégnation de ladite silice calcinée par la phase acide. Pour réaliser cette étape on peut utiliser toutes les techniques bien connues de l'homme du métier. A ce procédé de préparation peut s'ajouter une étape de préparation de la phase acide, préalable à l'étape d'imprégnation.

Le catalyseur selon l'invention ainsi préparé n'a subi aucune calcination postérieure à l'étape d'imprégnation. Lorsqu'il est utilisé en alkylation d'isoparaffine(s) par au moins une oléfine, il ne subit avant son utilisation aucune calcination et ainsi entre l'étape d'imprégnation et ladite utilisation, il n'a subi aucune calcination. Le catalyseur ainsi préparé est donc immédiatement prêt à l'emploi.

Le catalyseur selon la présente invention est mis en oeuvre dans un procédé qui permet de réaliser dans les meilleures conditions la réaction d'alkylation d'au moins une isoparaffine par au moins une oléfine. En particulier, ladite réaction étant caractérisée par une forte exothermicité (environ 83,6 kJ/mol de butène transformé), le procédé dans lequel est mis en oeuvre le catalyseur selon la présente invention permet d'obtenir une bonne homogénéité de température et de concentration en réactifs.

Dans le procédé d'alkylation de l'isoparaffine utilisant le catalyseur selon la présente invention, les conditions opératoires, et plus particulièrement la température et la pression, sont généralement choisies de façon à ce que le mélange constitué par l'(les) isoparaffine(s), l'(les) oléfine(s) et les produits de la réaction soit liquide. De plus, il est important que le catalyseur soit immergé dans ledit liquide de façon à assurer partout un bon contact liquide-solide.

Le catalyseur selon la présente invention est avantageusement mis en oeuvre dans la zone réactionnelle d'alkylation de l'isoparaffine avec au moins une oléfine comprenant de 3 à 6 atomes de carbone par molécule, en phase liquide et en mélange avec l'isoparaffine ou le mélange d'isoparaffines. Le catalyseur selon l'invention peut être mis en oeuvre en lit expansé, en zone réactionnelle presque parfaitement agitée ou en lit circulant, et de préférence il est mis en oeuvre dans un procédé qui utilise une phase liquide continue, le catalyseur pouvant être utilisé sous forme de suspension par exemple selon les deux mises en oeuvre décrites ci-après.

Dans le cas où le catalyseur est utilisé sous forme d'une suspension, on peut utiliser dans une première mise en oeuvre une zone réactionnelle à mélange presque parfait, c'est-à-dire à mélange parfait ou s'en rapprochant (cuve agité ou Grignard), utilisant au moins un moyen d'agitation par exemple au moins une hélice, de façon à obtenir une agitation suffisante du catalyseur en suspension dans la phase liquide hydrocarbonée, laquelle comprend généralement l'(les) isoparaffine(s) (isobutane et/ou isopentane), au moins une oléfine, éventuellement au moins un diluant inerte (par exemple le propane et le normal butane) et les produits de la réaction d'alkylation. La charge à convertir, constituée d'isobutane et/ou d'isopentane et d'au moins une oléfine, peut être par exemple introduite sous forme liquide en au moins un point au sein de la phase liquide hydrocarbonée présente dans la zone réactionnelle.

Une deuxième mise en oeuvre du catalyseur selon la présente invention en suspension dans une phase hydrocarbonée est le lit mobile à co-courant ou lit circulant. Dans cette mise en oeuvre le catalyseur en suspension dans la phase liquide hydrocarbonée, comprenant généralement l'isobutane et/ou l'isopentane, au moins une oléfine, éventuellement au moins un diluant inerte (par exemple le propane ou le normal butane) et les produits de la réaction d'alkylation, circule de bas en haut dans la zone réactionnelle. L'ensemble constitué par la suspension de catalyseur dans la phase hydrocarbonée circule ensuite au travers d'au moins un échangeur de chaleur et d'au moins une pompe, avant d'être de nouveau introduit à l'entrée de la zone réactionnelle. La charge à convertir, constituée d'isobutane et/ou d'isopentane et d'au moins une oléfine, est introduite soit sous forme liquide, soit sous forme gazeuse en au moins un point de la zone réactionnelle.

Dans les deux types de mises en oeuvre décrites précédemment, l'isoparaffine (isobutane et/ou isopentane) n'ayant pas été convertie ou ayant été introduite en excès par rapport à la stoechiométrie de la réaction, est généralement recyclée après séparation de l'alkylat, soit par introduction directe dans le réacteur soit par mélange avec la charge à convertir.

Le mélange isoparaffine(s)-oléfine(s) est généralement introduit dans la zone réactionnelle à une vitesse spatiale horaire, exprimée en poids d'oléfine introduite par unité de poids du catalyseur et par heure (pph) comprise entre 0,001 et 10 h⁻¹ et de préférence comprise entre 0,002 et 2 h⁻¹. Ledit mélange peut aussi être réalisé à l'intérieur de la zone réactionnelle. Dans tous les cas le mélange ainsi constitué est généralement dans la zone réactionnelle dans des conditions de pression et de température telles que le mélange d'hydrocarbures reste liquide sur le catalyseur.

La température de réaction est inférieure à +10 °C, de préférence à 0 °C et de manière souvent plus préférée inférieure à -3 °C. La pression de la zone réactionnelle est suffisante pour maintenir les hydrocarbures à l'état liquide dans ladite zone.

Afin de limiter les réactions secondaires, on peut utiliser un excès d'isoparaffine(s) par rapport à l'(les) oléfine(s). A titre d'exemple, dans le cas de l'alkylation de l'isobutane par un butène, l'isobutane peut être introduit pur dans la charge ou sous la forme d'un mélange de butanes contenant par exemple au moins 40 % d'isobutane. De plus, on peut introduire un butène pur ou encore un mélange de butènes isomères. Dans tous les cas, le rapport molaire isobutane/butène(s) dans la charge est généralement compris entre 1 et 100, de préférence entre 3 et 50 et de manière souvent préférée entre 5 et 15.

Lorsque la nature du catalyseur et les conditions de travail du catalyseur sont judicieusement choisies (en particulier la température), le catalyseur selon l'invention permet la production de produits d'alkylation d'au moins une isoparaffine par au moins une oléfine qui sont intéressants comme carburants pour les moteurs et constituants d'essence et qui comprennent par exemple au moins 60 % moles de paraffines possédant 8 atomes de carbone par molécule et moins de 1 % moles de composés non saturés, les paraffines comprenant 8 atomes de carbone par molécule comportant 70 à 98 % en moles de triméthylpentanes.

Un autre avantage du catalyseur selon la présente invention est la possibilité d'alkyler, à basse température, l'isobutane avec des mélanges d'oléfines comportant de 3 à 6 atomes de carbone par molécule, où la proportion d'oléfines comportant plus de 4 atomes de carbone par molécule est très importante.

Les exemples suivants illustrent l'invention sans toutefois en limiter la portée.

### EXEMPLE 1

### PRÉPARATION DU CATALYSEUR A CONFORME À L'INVENTION COMPORTANT DE L'ACIDE SULFURIQUE

On active 20 g de silice de volume poreux égal à 0,15 cm³/g, de diamètre moyen des particules égal à 18 µm et de surface spécifique égal à 210 m²/g, par séchage à 150° C pendant 12 heures. La silice ainsi activée est conservée sous argon. On réalise alors une imprégnation à sec de 20 g de ladite silice calcinée par 5,15 g d'acide sulfurique de titre pondéral égal à 100 %. Le solide obtenu, catalyseur A, possède une teneur pondérale en phase acide égale à 20,5 %. Il est conservé à l'abri de l'humidité et sous argon à - 18° C.

### ALKYLATION DE L'ISOBUTANE PAR LE BUTENE-1 AVEC LE CATALYSEUR A.

On introduit 20 g du catalyseur préparé selon la méthode décrite ci-dessus dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -20°C.
157 cm³ d'isobutane sont alors ajoutés dans le réacteur contenant le catalyseur sous agitation, ledit réacteur étant immergé dans un bain froid à -20° C. On laisse sous agitation le système catalyseur + isobutane pendant une durée de 30 minutes afin d'homogénéiser la température.
On ajoute régulièrement 2 g de butène-1 par heure pendant une durée totale de 6 heures, la température du réacteur est maintenue à -8,5° C pendant toute la durée de l'injection.
Après réaction, la phase hydrocarbure est soutirée du réacteur, puis l'isobutane est évaporé lentement et on recueille l'alkylat qui est analysé par chromatographie en phase vapeur. Sa composition pondérale est donnée dans le tableau 1 ci-après. La conversion de l'oléfine est de 100 %.

**TABLEAU 1**

| Composition alkylat (% poids) | Catalyseur A conforme à l'invention |
|---|---|
| % C₅-C₇ | 12,1 |
| % C₈ | 71,8 |
| % C₉⁺ | 16,1 |

### EXEMPLE 2 : PRÉPARATION D'UN CATALYSEUR B CONFORME À L'INVENTION COMPORTANT DE L'ACIDE SULFURIQUE ET DE L'ACIDE TRIFLUOROMETHANE SULFONIQUE

### PRÉPARATION DU CATALYSEUR

On active 30 g de silice de volume poreux total égal à 0,15 cm³/g, et de diamètre moyen de particules égal à 18 µm (1 µm = 10⁻⁶m) et de surface spécifique égale à 210 m²/g, par séchage à 150°C pendant 12 heures. La silice ainsi activée est conservée sous azote. On réalise alors une imprégnation à sec de 20 g de ladite silice calcinée par 5,15 g d'une solution d'acide contenant 86% en poids d'acide sulfurique de titre égal à 99,7 % en poids, et 14 % en poids d'acide trifluorométhane sulfonique de titre égal à 98 % en poids. Le solide obtenu, catalyseur B, possède une teneur pondérale en phase acide égale à 20,5 %. Il est conservé à l'abri de l'humidité.

### ALKYLATION DE L'ISOBUTANE PAR LE BUTENE-1

On introduit 20 g du catalyseur préparé selon la méthode décrite ci-dessus dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -20°C.
157 cm³ d'isobutane sont alors ajoutés dans le réacteur contenant le catalyseur sous agitation, ledit réacteur étant immergé dans un bain froid à -20° C. On laisse sous agitation le système catalyseur + isobutane pendant une durée de 30 minutes afin d'homogénéiser la température.
On ajoute régulièrement 2 g de butène-1 par heure pendant une durée totale de 6 heures, la température du réacteur est maintenue à -8,5° C pendant toute la durée de l'injection.
Après réaction, la phase hydrocarbure est soutirée du réacteur, puis l'isobutane est évaporé lentement et on recueille l'alkylat qui est analysé par chromatographie en phase vapeur. Sa composition pondérale est donnée dans le tableau 2 ci-après. La conversion de l'oléfine est de 100 %.

**TABLEAU 2**

| Composition alkylat (% poids) | Catalyseur B conforme à l'invention |
|---|---|
| C₅-C₇ | 10,2 |
| C₈ | 78,5 |
| C₉⁺ | 11,3 |

La fraction C₈ contient 89 % de triméthylpentanes.

## Revendications

1. Catalyseur comprenant de la silice et une phase acide comportant de l'acide sulfurique, la silice ayant été imprégnée par ladite phase acide et ledit catalyseur étant caractérisé en ce qu'il est constitué essentiellement de particules de diamètre moyen compris entre 0,1 et 30 µm, et en ce que la silice, avant son imprégnation par ladite phase acide, possède un volume poreux total compris entre 0,1 et 0,6 cm³/g.

2. Catalyseur selon la revendication 1 tel que le diamètre moyen des particules de catalyseur est compris entre 5 et 30 µm.

3. Catalyseur selon l'une des revendications 1 ou 2 tel que la teneur pondérale dudit catalyseur en phase acide est comprise entre 1 et 40 %.

4. Catalyseur selon l'une des revendications 1 à 3 tel que ladite phase acide comprend au moins un additif choisi dans le groupe formé par H₃PO₄, B(OH)₃, CF₃SO₃H, HB(HSO₄)₄, BF₄H, FSO₃H, CF₃CO₂H, SbF₅ et SO₃.

5. Catalyseur selon l'une des revendications 1 à 4 tel que ladite phase acide comprend de l'acide trifluorométhane sulfonique CF₃SO₃H et tel que la teneur pondérale de ladite phase en acide trifluorométhane sulfonique est comprise entre 0,1 et 50 %.

6. Utilisation du catalyseur selon l'une des revendications 1 à 5 dans un procédé d'alkylation catalytique d'au moins un élément choisi dans le groupe formé par l'isobutane et l'isopentane par au moins un élément choisi dans le groupe formé par les oléfines comprenant de 3 à 6 atomes de carbone par molécule, dans laquelle la température de la réaction est inférieure à 10° C.

7. Utilisation selon la revendication 6 dans laquelle le catalyseur est mis en oeuvre dans une zone réactionnelle à mélange partait ou s'en approchant.

8. Utilisation selon la revendication 6 dans laquelle le catalyseur est mis en oeuvre en lit mobile à co-courant.

## Claims

1. Catalyst containing silica and an acid phase consisting of sulphuric acid, the silica having been impregnated with said acid phase and said catalyst being such that it is essentially composed of particles with an average diameter of between 0.1 and 30 µm, and in that the silica, prior to its impregnation with said acid phase, has a total porous volume of between 0.1 and 0.6 cm³ per gram.

2. Catalyst according to claim 1 such that the average diameter of the particles of the catalyst is between 5 and 30 µm.

3. Catalyst according to one of claims 1 or 2 such that the content by weight of acid phase of said catalyst is between 1 and 40%.

4. Catalyst according to one of claims 1 to 3 such that said acid phase contains at least one additive selected from the group formed by H₃PO₄, B(OH)₃, CF₃SO₃H, HB(HSO₄)₄, BF₄H, FSO₃H, CF₃CO₂H, SbF₅ and SO₃.

5. Catalyst according to one of claims 1 to 4 such that said acid phase contains trifluoromethane sulphonic acid CF₃SO₃H and such that the content by weight of trifluoromethane sulphonic acid of said acid phase is between 0.1 and 50%.

6. Use of the catalyst according to one of claims 1 to 5 in a process of catalytic alkylation of at least one element selected from the group formed by isobutane and isopentane by at least one element selected from the group formed by olefins containing 3 to 6 carbon atoms per molecule, in which the reaction temperature is lower than 10°C.

7. Use according to claim 6 in which the catalyst is used in a perfect or near-perfect mixture reaction zone.

8. Use according to claim 6 in which the catalyst is used in a co-flow fluidised bed.

## Patentansprüche

1. Katalysator, der Siliciumoxid und eine saure Phase, die Schwefelsäure umfaßt, umfaßt, wobei das Siliciumoxid mit der sauren Phase getränkt ist, und der Katalysator dadurch gekennzeichnet ist, daß er im wesentlichen aus Partikeln mit einem mittleren Durchmesser zwischen 0,1 und 30 µm gebildet ist und daß das Siliciumoxid vor seiner Tränkung mit der sauren Phase ein Gesamtporenvolumen zwischen 0,1 und 0,6 cm³/g aufweist.

2. Katalysator nach Anspruch 1, wobei der mittlere Durchmesser der Partikel des Katalysators zwischen 5 und 30 µm ist.

3. Katalysator nach einem der Ansprüche 1 oder 2, wobei der Gewichtsgehalt des Katalysators an saurer Phase zwischen 1 und 40% beträgt.

4. Katalysator nach einem der Ansprüche 1 bis 3, wobei die saure Phase wenigstens ein Additiv, ausgewählt aus der durch H₃PO₄, B(OH)₃, CF₃SO₃H, HB(HSO₄)₄, BF₄H, FSO₃H, CF₃CO₂H, SbF₅ und SO₃ gebildeten Gruppe, umfaßt.

5. Katalysator nach einem der Ansprüche 1 bis 4, wobei die saure Phase Trifluormethansulfonsäure CF₃SO₃H umfaßt und wobei der Gewichtsgehalt der genannten Phase an Trifluormethansulfonsäure zwischen 0,1 und 50% beträgt.

6. Verwendung des Katalysators nach einem der Ansprüche 1 bis 5 in einem Verfahren der katalytischen Alkylierung wenigstens eines Elements, ausgewählt aus der durch Isobutan und Isopentan gebildeten Gruppe, mit wenigstens einem Element, ausgewählt aus der durch Olefine, die 3 bis 6 Kohlenstoffatome pro Molekül umfassen, gebildeten Gruppe, wobei die Reaktionstemperatur weniger als 10°C beträgt.

7. Verwendung nach Anspruch 6, wobei der Katalysator in einer Reaktionszone bei vollkommener Mischung oder dem nahekommend eingesetzt wird.

8. Verwendung nach Anspruch 6, wobei der Katalysator in einem Fließbett mit Gleichstrom eingesetzt wird.
